# EUROPEAN PATENT SPECIFICATION

(11) **EP 3 255 331 B1**
(45) Date of publication and mention of the grant of the patent: **31.03.2021**
(21) Application number: 17174666.2
(22) Date of filing: 07.06.2017
(51) Int. Cl.: F16L 23/10, F16L 23/22, A61M 39/18, F16J 15/06, A61M 39/12

(54) **A METHOD OF CONNECTING TWO CONDUITS OF EQUIPMENT TO BE STERILISED**
VERFAHREN ZUR VERBINDUNG VON ZWEI LEITUNGEN VON ZU STERILISIERENDER AUSRÜSTUNG
PROCÉDÉ PERMETTANT DE RACCORDER DEUX CONDUITS D'ÉQUIPEMENT À STÉRILISER

(30) Priority: 07.06.2016 NL 2016906
(43) Date of publication of application: 13.12.2017
(73) Proprietor: ULTRAPHARMA HOLDING BV, 1851 LX Heiloo (NL)
(72) Inventor: SEPPENWOOLDE, Hendrikus Antoon, 2071 ET Santpoort Noord (NL)
(74) Representative: Altenburg, Bernardus Stephanus Franciscus

(56) References cited:
- WO-A1-2009/146764
- WO-A1-2010/144500
- US-A- 3 042 430
- US-A- 4 438 960
- US-A1- 2011 254 268

## Description

The present invention relates to a method of connecting two conduits of equipment to be sterilised, each conduit comprising at a free end thereof a ferrule, wherein a gasket comprising a through-hole is provided between the ferrules of the conduits and a clamp is used to clamp the ferrules together, said clamp comprising two arms, each arm comprising a recess for receiving a circumferential section of the ferrules;
wherein in a state wherein the ferrules are clamped by the clamp, the ferrules provide a passage and the inner surface of the through-hole of the gasket after clamping is flush with the inner wall of the passage provided by the ferrules, and the gasket is capable of bulging into said passage by a temporary increase in temperature.

It is known in the art to connect two conduits each comprising a ferrule. A ferrule comprises a flange that tapers to a reduced thickness towards the circumference of the flange. A gasket is provided between the two opposing parallel first surfaces of the flanges. The gasket is a disk of elastomeric (resilient) material, such as EPDM, and has a through-hole with a diameter that is slightly larger than the inner diameter of the ferrules. By clamping the ferrules together by pressing the clamp against second surfaces of the flanges that provide the taper, the gasket is somewhat compressed. As a result of the compression of the gasket, the diameter of the through-hole of the gasket is reduced. The objective is that when the ferrules have been clamped together, the through-hole of the gasket has the same diameter as the inner diameter of the ferrules, i.e. the inner surface of the through-hole of the gasket after clamping is flush with the inner wall of the passage provided by the ferrules. This helps to avoid that dirt accumulates at the location of the gasket, and also the flow resistance of liquid passed through the connection is not affected, i.e. there is no pressure drop at the joint. The latter may be in particular significant in case there are several connections, each with a pressure drop.

The above method is in use in biotech plants and in particular in pharmaceutical plants. There it is often required to sterilise the equipment containing the connection provided by the two clamped ferrules. To this end steam is passed through the conduits, e,g. at 121°C and about 2 Bar steam pressure. As a result of the resulting thermal expansion, the gasket is compressed even more and bulges into the lumen of the connection. Being resilient material, it should return to its original shape once the temperature is back to the regular operating temperature, but it has been found that this is often not the case. As a result, the edge of the hole of the gasket protruding into the lumen of the connection results in a pressure drop. In addition, protruding material of the gasket is vulnerable and may break off, which is undesirable. The problem of bulging, which increases with the number of sterilisation operations, exists even if a torque wrench is used to close the clamp to the intended strength so as to have inner surface of the through-hole of the gasket the flush with the inside of the conduits, the torque wrench preventing over tightening which could exacerbate the problem.

The object of the present invention is to provide a method in which the problem of gasket bulging caused by a temporary increase in temperature, such as in case of sterilisation, is reduced.

To this end, a method according to the preamble is characterized in that a resilient element is provided in the recess of at least one arm of the clamp between said at least one arm and the ferrules, wherein the resilient element is an elastomeric lining, wherein the recess of at least one arm of the clamp comprises said elastomeric lining.

For gaskets that have even been subjected to multiple heat cycles (steam sterilisation at 121°C and about 2 Bar)) it has surprisingly been found that the damage is reduced or even absent.

The thickness of the elastomeric element will in general be at least 0.3 mm. The elastomeric element is for example made of EPDM rubber. It is for example a rubber band, which is preferably wider than the combined width of the circumferential edges of the flanges of the ferrules and the gasket. Thus, at least part of the rubber band will be in contact with a second surface of a flange of a ferrule.

An arm of a clamp used in the method according to the invention may be an articulated arm.

US3828403 discloses a clamp for sealing the circumference of a joint using an elastomeric material.

US3432189A discloses a method of joining plain-ended pipes by providing a sleeve over both pipe ends.

GB552080A discloses a clamp for providing a compression joint, said clamp comprising an elastic ring.

US5380052A discloses a clamp comprising two identical arms.

WO2009146764 discloses a clamp for joining two pipe ends comprising ferrules with a gasket in between.

US2011254268 discloses a clamp for connecting cooling hoses to an engine of an automotive vehicle.

None of these publications disclose or suggest that a problem caused by bulging of a gasket between the ferrules can be reduced with a clamp comprising a resilient element.

According to a favourable embodiment, the thickness of the elastomeric lining is at least 0.7 mm, preferably at least 1 mm.

This has been found to give both excellent performance with respect to reduction of the problem, and provides good sealing against leakage of liquid at the connection for most usage conditions as seen in practice.

According to a favourable embodiment, the thickness of the elastomeric lining is less than 10 mm, preferably less than 6 mm.

This has been found to give both excellent performance with respect to reduction of the problem, and still provides good sealing against leakage of liquid at the connection for most usage conditions as seen in practice.

According to a favourable embodiment, the elastomer is silicone.

Silicone was found to have excellent suitability for preventing damage to gaskets.

According to a favourable embodiment, the clamp comprising two arms is a clamp comprising a first arm and a second arm, each of said arms comprising a first end section and a second end section and between said first end section and said second end section a third section, said third section comprising
- a first wall section extending between the first end section and the second end section,
- a second wall section extending between the first end section and the second end section, and
- a third connecting section connecting the first wall section and the second wall section; a first face of the first wall section, a first face of the third connecting section and a first face of the second wall section defining a groove, wherein the first face of the first wall section and the first face of the second wall section defining the groove as tapering towards the first face of the third connecting section,
   wherein
   at least one of the first end sections is releasably connectable
   - to lock the clamp in a first closed state, and
   - to open the clamp defining a second state of the clamp; wherein of at least one arm at least one of i) the first face of the first wall section, and ii) the first face of the second wall section comprises the elastomeric lining.

Use of such a clamp for clamping an assembly comprising ferrules and a gasket has been found to result in less damage to the gasket when the assembly is subjected to frequent temporary elevated temperatures such as occurring during sterilisation.

Preferably both first faces of the wall sections are provided with the elastomeric lining.

Preferably both arms are provided with the elastomeric lining.

The elastomeric lining is preferably silicone.

An arm of a clamp according to the invention may be an articulated arm.

According to a favourable embodiment, the first end section and the second end section are connected using a hinge.

By being hingeably connected, the use of separate arms can be avoided, making the clamp easier to handle.

According to a favourable embodiment, the method further comprising the step of sterilising the equipment.

This is the ultimate purpose of the invention, and in particular allowing repeated sterilisation operations with a reduced risk of the gasket not returning properly when the temperature is reduced after sterilisation.

According to a favourable embodiment, the sterilisation is performed using steam.

The present invention will now be illustrated with reference to the drawing where
Fig. 1A to Fig. 1D show perspective views of a method of connecting two ferrules of two conduits; and
Fig. 2A and Fig. 2B show cross-sectional views through an assembly of two ferrules and a gasket, without a clamp and with a clamp respectively.

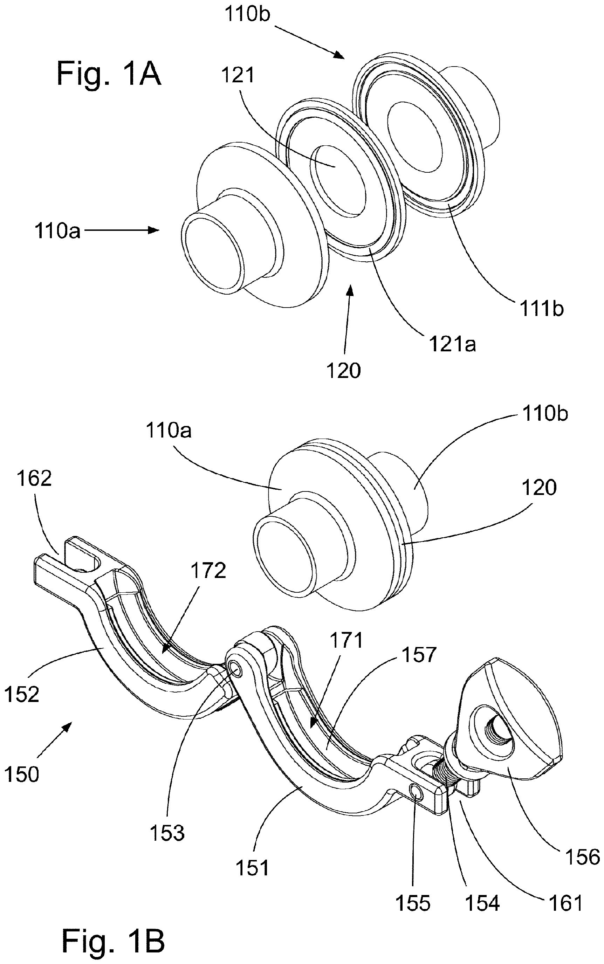

Fig. 1A to Fig. 1D show perspective views of a method of connecting two ferrules (ferrule 110a and ferrule 110b) of conduits (not shown for reasons of clarity) using a clamp 150 according to the invention.

The two ferrules 110a, 110b are separated by a gasket 120 having a central hole 121 having a diameter that is slightly larger than the inner diameter of the ferrules 110a, 110b. In addition, the gasket 120 comprises at both faces thereof an annular ridge (ridge 121a is visible in Fig. 1A) that is received in a corresponding annular recess (annular recess 111b is visible in Fig. 1A) of the ferrules. This helps to center the gasket 120 between the ferrules 110a, 110b and thus to center the hole 121 (Fig. 1B).

A clamp 150 is used to hold the assembly of the ferrules 110a, 110b and the gasket 120 together. In the embodiment shown, the clamp 150 comprises a first arm 151 and a second arm 152. The arms of a clamp may be separate pieces, but in the embodiment discussed here they are capable of hinging at first ends of said arms 151, 152 at hinge 153. The arms 151, 152 are typically made of metal. In the art such clamping arms are also referred to as shells or clamping members. The second end of the first arm 151 is forked comprising two prongs to provide a first aperture 161 that is provided with a threaded rod 154 capable of hinging about pin 155 held by the prongs at a first end thereof. The threaded rod 154 is provided with a nut 156 for tightening the clamp 150.

The second arm 152 also has a forked second end comprising two prongs providing a second aperture 162 for receiving the threaded rod 154 and to provide a contact surface for the nut 156 when the clamp 150 is tightened.

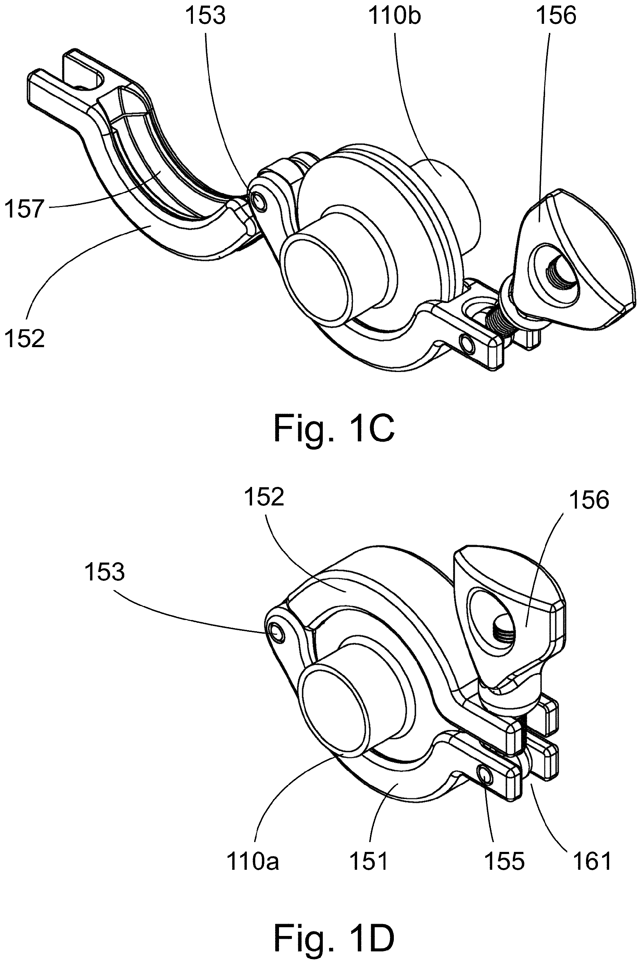

The first arm 151 comprises a first groove 171 and the second arm 152 comprises a second groove 172 as recesses for receiving part of the circumference of the assembly of both ferrules 110a, 110b and the gasket 120 in a closed state of the clamp (the closed state is shown Fig 1D).

As described so far, this is a normal clamp.

The clamp 150 according to the present invention comprises an elastomeric lining 157, here of silicone having a thickness of 1.5 mm.

Fig. 1C shows that the assembly of both ferrules 110a, 110b and the gasket 120 is received in the first groove 171 of the first arm 151 over part of the circumference thereof, in contact with the elastomeric lining 157.

To clamp the assembly of both ferrules 110a, 110b and the gasket 120 together the second arm 152 is rotated using the hinge 153, the threaded rod 154 is pivoted so as to receive it in the second aperture 162 of the second arm 152 and the nut 156 is tightened (Fig. 1D).

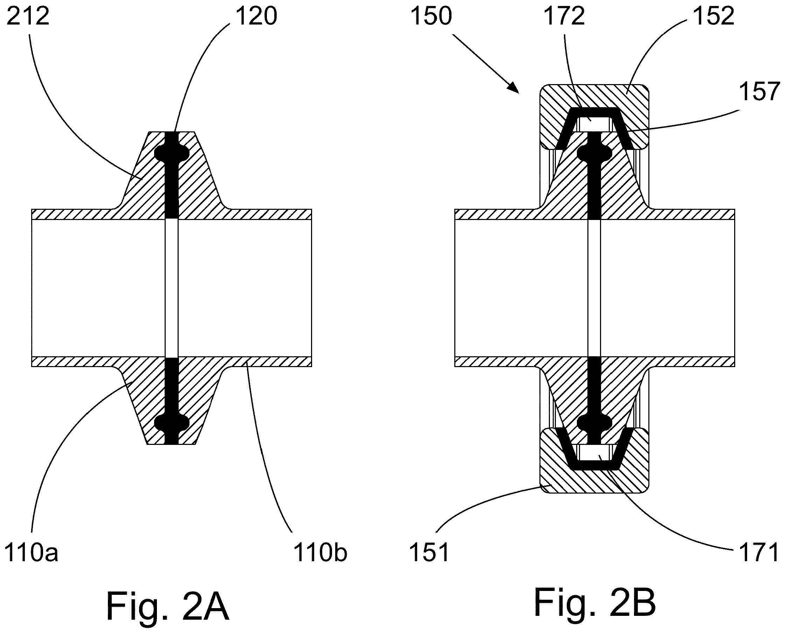

Fig. 2A and Fig. 2B show a cross-sectional view through an assembly of two ferrules 110a, 110b and a gasket 120 without a clamp 150 and with a clamp 150 respectively.

It can be seen that in Fig. 2A before the clamp 150 is applied the diameter of the hole 121 of gasket 120 is somewhat larger than the inner diameter of the ferrules 110a, 110b. By tightening the clamp (Fig. 2B), the gasket 120 which is made of an elastomeric material such as EPDM is compressed and the diameter of the hole 121 is reduced accordingly such that it is flush with the inner walls of the ferrules 110a, 110b.

Fig. 2A and Fig. 2B show that a ferrule 110 comprises a flange 212 that tapers to a reduced thickness towards the circumference of the flange 212. Similarly, the grooves provided with lining 157 of the arms are also tapered towards the circumference of the closed clamp 150. It has been found that when the clamped assembly as shown in Fig. 2B is subjected to elevated temperature and pressure repeatedly (e.g. as is necessary during sterilisation with steam), the gasket 120 that is compressed even further during such an operation causing the diameter of the hole 121 to be reduced even further (effectively bulging into the passage provided by the assembly) will return to the original (flush) state reliably and repeatedly. Thus, equipment can be operated for a longer period of time with reduced maintenance. The risk of debris piling up at the joint is reduced. Also, the risk of gasket material ending up downstream and possibly in the product is reduced.

## Claims

1. A method of connecting two conduits of equipment to be sterilised, each conduit comprising at a free end thereof a ferrule, wherein a gasket (120) comprising a through-hole is provided between the ferrules (110a, 110b) of the conduits and a clamp (150) is used to clamp (150) the ferrules (110a, 110b) together, said clamp (150) comprising two arms (151, 152), each arm comprising a recess (171, 172) for receiving a circumferential section of the ferrules (110a, 110b);
wherein in a state wherein the ferrules (110a, 110b) are clamped by the clamp (150), the ferrules (110a, 110b) provide a passage and the inner surface of the through-hole of the gasket (120) after clamping is flush with the inner wall of the passage provided by the ferrules (110a, 110b), and the gasket (120) is capable of bulging into said passage by a temporary increase in temperature;
**characterized in that** a resilient element (157) is provided in the recess (171, 172) of at least one arm of the clamp (150) between said at least one arm and the ferrules (110a, 110b), wherein the resilient element (157) is an elastomeric lining (157), wherein the recess (171, 172) of at least one arm of the clamp (150) comprises said elastomeric lining.

2. The method according to claim 1, wherein the thickness of the elastomeric lining is at least 0.7 mm, preferably at least 1 mm.

3. The method according to claim 1 or 2, wherein the thickness of the elastomeric lining is less than 10 mm, preferably less than 6 mm.

4. The method according to any of the preceding claims, wherein the elastomer is silicone.

5. The method according to any of the preceding claims, wherein the clamp (150) comprising two arms (151, 152) is a clamp (150) comprising a first arm (151) and a second arm (152), each of said arms (151, 152) comprising a first end section and a second end section and between said first end section and said second end section a third section, said third section comprising
- a first wall section extending between the first end section and the second end section,
- a second wall section extending between the first end section and the second end section, and
- a third connecting section connecting the first wall section and the second wall section; a first face of the first wall section, a first face of the third connecting section and a first face of the second wall section defining a groove,
wherein the first face of the first wall section and the first face of the second wall section defining the groove as tapering towards the first face of the third connecting section,
wherein
at least one of the first end sections is releasably connectable
- to lock the clamp (150) in a first closed state, and
- to open the clamp (150) defining a second state of the clamp (150);
wherein of at least one arm (151, 152) at least one of i) the first face of the first wall section, and ii) the first face of the second wall section comprises the elastomeric lining (157).

6. The method according to claim 5, wherein the first end section and the second end section are connected using a hinge (153).

7. The method according to any of the preceding claims, wherein the method further comprising the step of sterilising the equipment.

8. The method according to claim 7, wherein the sterilisation is performed using steam.

## Patentansprüche

1. Verfahren zum Verbinden zweier Leitungen einer zu sterilisierenden Ausrüstung, wobei jede Leitung an einem freien Ende derselben eine Hülse umfasst, wobei eine Dichtung (120), die ein Durchgangsloch umfasst, zwischen den Hülsen (110a, 110b) der Leitungen bereitgestellt ist und eine Klemme (150) zum Zusammenklemmen (150) der Hülsen (110a, 110b) verwendet wird, wobei die Klemme (150) zwei Arme (151, 152) umfasst, wobei jeder Arm eine Aussparung (171, 172) zur Aufnahme eines Umfangsabschnitts der Hülsen (110a, 110b) umfasst;
wobei in einem Zustand, in dem die Hülsen (110a, 110b) durch die Klemme (150) festgeklemmt sind, die Hülsen (110a, 110b) einen Durchgang bereitstellen und die innere Oberfläche des Durchgangslochs der Dichtung (120) nach dem Festklemmen bündig mit der Innenwand des von den Hülsen (110a, 110b) bereitgestellten Durchgangs ist, und die Dichtung (120) in der Lage ist, sich durch einen vorübergehenden Temperaturanstieg in den Durchgang hinein auszuwölben,
**dadurch gekennzeichnet, dass** ein elastisches Element (157) in der Aussparung (171, 172) von mindestens einem Arm der Klemme (150) zwischen dem mindestens einen Arm und den Hülsen (110a, 110b) vorgesehen ist, wobei das elastische Element (157) eine elastomere Auskleidung (157) ist, wobei die Aussparung (171, 172) von mindestens einem Arm der Klemme (150) die elastomere Auskleidung umfasst.

2. Verfahren nach Anspruch 1, wobei die Dicke der elastomeren Auskleidung mindestens 0,7 mm, vorzugsweise mindestens 1 mm, beträgt.

3. Verfahren nach Anspruch 1 oder 2, wobei die Dicke der elastomeren Auskleidung weniger als 10 mm, vorzugsweise weniger als 6 mm, beträgt.

4. Verfahren nach einem der vorhergehenden Ansprüche, wobei das Elastomer Silikon ist.

5. Verfahren nach einem der vorstehenden Ansprüche, wobei die zwei Arme (151, 152) umfassende Klemme (150) eine Klemme (150) ist, die einen ersten Arm (151) und einen zweiten Arm (152) umfasst, wobei jeder der Arme (151, 152) einen ersten Endabschnitt und einen zweiten Endabschnitt und zwischen dem ersten Endabschnitt und dem zweiten Endabschnitt einen dritten Abschnitt umfasst, wobei der dritte Abschnitt umfasst
- einen ersten Wandabschnitt, der sich zwischen dem ersten Endabschnitt und dem zweiten Endabschnitt erstreckt
- einen zweiten Wandabschnitt, der sich zwischen dem ersten Endabschnitt und dem zweiten Endabschnitt erstreckt, und
- einen dritten Verbindungsabschnitt, der den ersten Wandabschnitt und den zweiten Wandabschnitt verbindet; eine erste Fläche des ersten Wandabschnitts, eine erste Fläche des dritten Verbindungsabschnitts und eine erste Fläche des zweiten Wandabschnitts, die eine Nut definieren,
wobei die erste Fläche des ersten Wandabschnitts und die erste Fläche des zweiten Wandabschnitts, die die Nut definieren, sich in Richtung der ersten Fläche des dritten Verbindungsabschnitts verjüngen,
wobei mindestens einer der ersten Endabschnitte lösbar verbindbar ist
- um die Klemme (150) in einem ersten geschlossenen Zustand zu verriegeln, und
- um die Klemme (150), die einen zweiten Zustand der Klemme (150) definiert, zu öffnen;
wobei von mindestens einem Arm (151, 152) mindestens einer von i) der ersten Fläche des ersten Wandabschnitts und ii) der ersten Fläche des zweiten Wandabschnitts die elastomere Auskleidung (157) umfasst.

6. Verfahren nach Anspruch 5, wobei der erste Endabschnitt und der zweite Endabschnitt unter Verwendung eines Scharniers (153) verbunden sind.

7. Verfahren nach einem der vorhergehenden Ansprüche, wobei das Verfahren weiter den Schritt des Sterilisierens der Ausrüstung umfasst.

8. Verfahren nach Anspruch 7, wobei die Sterilisation unter Verwendung von Dampf durchgeführt wird.

## Revendications

1. Procédé destiné à relier deux conduits d'un équipement qui doit être stérilisé, chaque conduit comprenant une bague à une extrémité libre dudit conduit ; dans lequel un joint d'étanchéité statique (120) qui comprend un trou de passage est prévu entre les bagues (110a, 110b) des conduits et un collier (150) est utilisé pour le serrage (150) des bagues (110a, 110b) l'une contre l'autre, ledit collier (150) comprenant deux bras (151, 152), chaque bras comprenant un évidement (171, 172) destiné à la réception d'un tronçon circonférentiel des bagues (110a, 110b) ;
dans lequel, dans un état dans lequel les bagues (110a, 110b) sont serrées par le collier (150), les bagues (110a, 110b) procurent un passage et la surface interne du trou de passage du joint d'étanchéité statique (120), après le serrage, est disposé à fleur avec la paroi interne du passage procuré par les bagues (110a, 110b), et le joint d'étanchéité statique (120) est capable de subir un gonflement à l'intérieur dudit passage par l'intermédiaire d'une élévation temporaire de la température ;
**caractérisé en ce que** l'on prévoit un élément résilient (157) dans l'évidement (171, 172) d'au moins un bras du collier (150) entre ledit au moins un bras et les bagues (110a, 110b) ; dans lequel l'élément résilient (157) représente un revêtement intérieur élastomère (157) ; dans lequel l'évidement (171, 172) d'au moins un bras du collier (150) comprend ledit revêtement intérieur élastomère.

2. Procédé selon la revendication 1, dans lequel l'épaisseur du revêtement intérieur élastomère s'élève à au moins 0,7 mm, de préférence à au moins 1 mm.

3. Procédé selon la revendication 1 ou 2, dans lequel l'épaisseur du revêtement intérieur élastomère est inférieure à 10 mm, de préférence inférieure à 6 mm.

4. Procédé selon l'une quelconque des revendications précédentes, dans lequel l'élastomère représente du silicone.

5. Procédé selon l'une quelconque des revendications précédentes, dans lequel le collier (150) comprenant deux bras (151, 152) représente un collier (150) qui comprend un premier bras (151) et un deuxième bras (152), chacun desdits bras (151, 152) comprenant un premier tronçon terminal et un deuxième tronçon terminal et, entre ledit premier tronçon terminal et ledit deuxième tronçon terminal, un troisième tronçon, ledit troisième tronçon comprenant :
- un premier tronçon faisant office de paroi qui s'étend entre ledit premier tronçon terminal et ledit deuxième tronçon terminal ;
- un deuxième tronçon faisant office de paroi qui s'étend entre ledit premier tronçon terminal et ledit deuxième tronçon terminal ; et
- un troisième tronçon de liaison qui relie le premier tronçon faisant office de paroi et le deuxième tronçon faisant office de paroi ; une première face du premier tronçon faisant office de paroi, une première face du troisième tronçon de liaison et une première face du deuxième tronçon faisant office de paroi définissant une rainure ; dans lequel la première face du premier tronçon faisant office de paroi et la première face du deuxième tronçon faisant office de paroi définissent la rainure sous la forme d'un rétrécissement qui s'étend dans la direction de la première face du troisième tronçon de liaison ;
dans lequel
au moins un desdits premiers tronçons terminaux peut être relié de manière amovible :
- dans le but de verrouiller le collier (150) dans un premier état fermé ; et
- dans le but d'ouvrir le collier (150) en définissant un deuxième état du collier (150) ;
dans lequel, en ce qui concerne au moins un bras (151, 152), au moins une première face choisie parmi i) la première face du premier tronçon faisant office de paroi et ii) la première face du deuxième tronçon faisant office de paroi comprend le revêtement intérieur élastomère (157).

6. Procédé selon la revendication 5, dans lequel le premier tronçon terminal et le deuxième tronçon terminal sont reliés en utilisant une articulation (153).

7. Procédé selon l'une quelconque des revendications précédentes, dans lequel le procédé comprend en outre l'étape dans laquelle on stérilise l'équipement.

8. Procédé selon la revendication 7, dans lequel la stérilisation est mise en œuvre en utilisant de la vapeur.
